# EUROPÄISCHE PATENTANMELDUNG

(11) **EP 0 635 281 A1**
(43) Veröffentlichungstag der Anmeldung: **25.01.1995**
(21) Anmeldenummer: 94108229.9
(22) Anmeldetag: 27.05.1994
(51) Int. Cl.: A61N 1/08, G01M 3/20

(54) **Anordnung zum Suchen von Leckstellen bei einer Elektrodenvorrichtung**

(30) Priorität: 09.07.1993 SE 9302385
(71) Anmelder: Pacesetter AB, S-171 95 Solna (SE)
(72) Erfinder: Lindegren, Ulf, S-122 35 Enskede (SE); Neubauer, Heinz, S-175 70 Järfälla (SE); Strandberg, Hans, S-172 36 Sundbyberg (SE); Schüller, Hans, S-224 67 Lund (SE)
(74) Vertreter: Lettström, Richard Wilhelm

(57) **Zusammenfassung**

Die Erfindung bezieht sich auf eine Anordnung zum Suchen von Leckstellen bei einer Elektrodenvorrichtung, insbesondere einer Herzschrittmacher- oder einer Defibrillierungselektrode, bestehend aus einem Elektrodenkabel mit einem langgestreckten flexiblen Leiter, dessen Aussenseite mit einer Isolierschicht versehen ist und dessen Innenseite einen Kanal bildet. Um eine Anordnung zum Suchen von Leckstellen zu erhalten, die im Aufbau einfach und dadurch in der Herstellung billig ist und bei der mit Hilfe der Anordnung in extrem einfacher Weise eine oder mehrere Leckstellen bei einer in einem Patienten implantierten Elektrodenvorrichtung angezeigt werden können, wird erfindungsgemäss vorgeschlagen, dass die Anordnung ein rohrförmiges flexibles Organ (2, 14) ist, dessen Aussendurchmesser kleiner als der Innendurchmesser des Kanals (10) ist und das in den Kanal (10) des Elektrodenkabels (6) bis zum distalen Ende einschiebbar ist, und dass das proximale Ende des Organs (2, 14) an einem Behälter (3) für Flüssigkeit (5) angeschlossen ist, der einen Überdruck erzeugen kann, so dass die Flüssikgeit (5) durch das Organ (2, 14) und in den Kanal (10) transportiert wird und somit den Kanal (10) derart füllen kann, dass ein radialer Flüssigkeitsdruck im Kanal (10) entsteht, der höher als der das Elektrodenkabel (6) umgebende Druck ist.

## Beschreibung

Die Erfindung bezieht sich auf eine Anordnung zum Suchen von Leckstellen bei einer Elektrodenvorrichtung, insbesondere einer Herzschrittmacher- oder eine Defibrillierungselektrode, bestehend aus einem Elektrodenkabel mit einem langgestreckten flexiblen Leiter, dessen Aussenseite mit einer Isolierschicht versehen ist und dessen Innenseite einen Kanal bildet.

Das Suchen von Leckstellen bei Elektrodenvorrichtungen, die nicht in einem Patienten implantiert sind, erfolgt mit Hilfe einer Druckluftvorrichtung, die an den Eingang des Kanals des Elektrodenkabels angeschlossen wird. Danach wird Luft in den Kanal hineingepresst, so dass ein Überdruck erzeugt wird. Danach wird das Elektrodenkabel in ein Wasserbad hineingesenkt, wobei bei einem defekten Elektrodenkabel Luft vom Kanal durch die Leckstelle bzw. die Leckstellen herausströmt, so dass Luftblasen im Wasser, die die Leckstellen anzeigen, entstehen.

Wenn bei einem implantierten Herzschrittmachersystem, bestehend aus einem Herzschrittmacher und einer an diesen angeschlossenen Herzschrittmacherelektrode, z.B. schlechte Schwellenwerte oder sinkende Impedanzwerte entstehen, kann der Arzt daraus schliessen, dass dies von einem defekten Herzschrittmacher oder von einer Leckstelle am Elektrodenkabel stammt, durch die Körperflüssigkeit in den Kanal eindringen und dadurch mit einem eventuell nicht isolierten Leiter in Verbindung kommen kann. Eine derartige Leckstelle kann in Verbindung mit einer Einführung einer Herzschrittmacherelektrode in das Herz eines Patienten über eine Vene entstehen. Eine derartige Einführung erfolgt in der Regel mit Hilfe eines verhältnismässig steifen Mandrins. Es ist nicht auszuschliessen, dass der Mandrin bei der Einführung in den Kanal des Elektrodenkabels oder bei einer Verschiebung des Mandrins in dem Kanal nicht nur den Leiter, der in der Regel den Kanal bildet, durchdringt, sondern auch die Isolierschicht penetriert. Eine Leckstelle kann auch beim Ermüden oder beim Abnutzen des Isoliermaterials entstehen. Bei einer Durchleuchtung einer implantierten Herzschrittmacherelektrode kann ein erfahrener Arzt einen Bruch an der Leitung entdecken. Es ist dagegen nicht möglich, eine Leckstelle an der Isolierschicht selbst zu entdecken. Nachdem der Arzt festgestellt hat, dass der Fehler nicht im Herzschrittmacher liegt, hat er bis heute die implantierte Herzschrittmacherelektrode durch eine neue ersetzt, ohne eigentlich zu wissen, ob die Isolierschicht des Elektrodenkabels defekt war. Eine solche Feststellung war auch dann nicht möglich, wenn der Arzt die Elektrodenvorrichtung explantiert hat, da das Kabel in der Regel in der Explantationsphase beschädigt wurde.

Der Erfindung liegt die Aufgabe zugrunde, eine Anordnung zum Suchen von Leckstellen bei einer Elektrodenvorrichtung der eingangs genannten Art zu schaffen, bei der die Suchanordnung im Aufbau einfach und dadurch in der Herstellung billig ist und bei der mit Hilfe der Suchanordnung in einer extrem einfachen Weise eine oder mehrere Leckstellen bei einer in einem Patienten implantierten Elektrodenvorrichtung angezeigt werden können.

Diese Aufgabe ist erfindungsgemäss dadurch gelöst, dass die Anordnung ein rohrförmiges flexibles Organ ist, dessen Aussendurchmesser kleiner als der Innendurchmesser des Kanals ist und dass in den Kanal des Elektrodenkabels bis zum distalen Ende einschiebbar ist, und dass das proximale Ende des Organs an einem Behälter für Flüssigkeit angeschlossen ist, der einen Überdruck erzeugen kann, so dass die Flüssigkeit durch das Organ und in den Kanal transportiert wird und somit den Kanal derart füllen kann, dass ein radialer Flüssigkeitsdruck im Kanal entsteht, der höher als der das Elektrodenkabel umgebende Druck ist. Wenn nun eine oder mehrere Leckstellen in der Isolierung des Elektrodenkabels vorhanden sind, wird dies angezeigt, indem die Flüssigkeit in dem Kanal, die eine Art von Kontrastmittel ist, durch die Leckstelle bzw. die Leckstellen heraussickert. Da eine solche Untersuchung in Verbindung mit einer Durchleuchtung der Brust eines Patienten durchgeführt wird, kann der Arzt, der den Verlauf der Leckstellenuntersuchung an einem Bildschirm verfolgt, teils das Vorkommen von Leckstellen und, falls solche vorhanden sind, auch deren Ort registrieren.

In einer vorteilhaften Weiterbildung der Erfindung wird vorgeschlagen, dass der Aussendurchmesser des rohrförmigen, flexiblen Organs derart bemessen ist, dass bei einem in dem Kanal eingeschobenen Organ Raum zwischen der Aussenwand des Organs und der Innenwand des Kanals gebildet wird, der mit der Flüssigkeit gefüllt werden kann.

Durch die Erfindung ist auch ein Verfahren zur Durchführung eines Suchens von Leckstellen bei einer Elektrodenvorrichtung mit einer Anordnung nach der erwähnten vorteilhaften Ausführungsform geschaffen, indem das Organ in den Kanal bis zu dessen distalem Ende hineingeschoben und die Flüssigkeit danach den Kanal und den Raum zwischen dem Organ und der Innenwand des Kanals derart füllt, dass ein radialer Flüssigkeitsdruck im Raum entsteht, der höher als der das Elektrodenkabel umgebende Druck ist. Auf diese Weise kann eine schnelle Füllung des Kanals des Elektrodenkabels mit einer geringen Menge Kontrastflüssigkeit, erhalten werden. Es ist von grosser Bedeutung, dass das Organ bis zum distalen Ende in den Kanal hineingeschoben wird. Hierdurch wird vermieden, dass Luftblasen und eventuelle Schmutzpartikel im Kanal durch eine eventuelle Leckstelle an der Isolierschicht in den Körper herausgedrückt werden, was für den Patienten schädlich sein kann. Die Luft und die eventuellen Schmutzpartikel werden stattdessen mit der steigenden Flüssigkeitssäule im Kanal hochgedrückt und aus dem Kanal herausgedrückt.

In einer vorteilhaften Ausbildung der Erfindung wird vorgeschlagen, dass der Aussendurchmesser des rohrförmigen, flexiblen Organs derart bemessen ist, dass es bei einem im Kanal eingeschobenen Organ nicht möglich ist, dass Flüssigkeit in den Spalt zwischen der Aussenwand des Organs und der Innenwand des Kanals eindringt.

Durch die Erfindung ist auch ein Verfahren zur Durchführung eines Suchens von Leckstellen bei einer Elektrodenvorrichtung mit einer Anordnung nach der erwähnten zweiten vorteilhaften Ausführungsform gegeben, indem das Organ in den Kanal bis zu dessen distalen Ende hineingeschoben wird und dass die Flüssigkeit danach in den Kanal transportiert wird, wobei das Organ nach hinten verschoben wird gleichzeitig damit, dass der Kanal vor der Stirnseite des Organs nach und nach mit Flüssigkeit gefüllt wird, wobei die Füllung derart erfolgt, dass ein radialer Flüssigkeitsdruck im Kanal entsteht, der höher als der das Elektrodenkabel umgebende Druck ist. Durch das beschriebene Verfahren kann der Arzt auf ganz einfache Weise die Flüssigkeitssäule im Kanal begrenzen gleichzeitig damit, dass er den entsprechenden Teil der Isolierschicht des Elektrodenkabels in Bezug auf eventuelle Leckstellen untersuchen kann. Die im Kanal vorhandene Luft und eventuelle Schmutzpartikel drängen nach und nach mit dem Hineintransportieren der Flüssigkeit in den Kanal zwischen der Aussenwand des Organs und der Innenwand des Kanals heraus.

Im Hinblick auf eine vorteilhafte Ausgestaltung der Erfindung wird vorgeschlagen, dass der Behälter für die Flüssigkeit eine Spritze ist, mit der die Flüssigkeit über das Organ in den Kanal hineingedrückt wird. Hierdurch wird erreicht, dass der Arzt die Flüssigkeitsmenge, die in den Kanal hineintransportiert werden soll, in einfacher Weise dosieren kann.

Die Erfindung ist nachfolgend anhand eines in den Zeichnungen dargestellten Ausführungsbeispiels näher erläutert. Es zeigen:
- FIG 1: eine Anordnung zum Suchen von Leckstellen gemäss der Erfindung, die hier in einer Elektrodenvorrichtung hineingeführt ist und
- FIG 2-5: Seitenansichten des distalen Endes der Elektrodenvorrichtung nach FIG 1 in einem Längsschnitt mit darin angeordneten Anordnungen zum Suchen von Leckstellen gemäss der Erfindung in verschiedenen Lagen und Ausführungsformen.

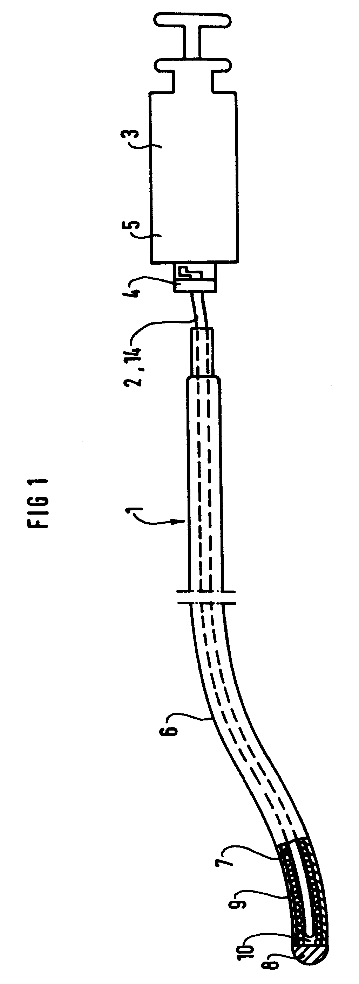

In der FIG 1 ist eine Anordnung zum Suchen von Leckstellen bei einer Elektrodenvorrichtung 2, z.B. eine Herzschrittmacherelektrode abgebildet, bestehend aus einem rohrförmigen flexiblen Organ 2, dessen proximales Ende mit Hilfe eines Schnellverschlusses 4 an einer Spritze 3 angeschlossen ist. Die Spritze 3 ist mit Kontrastflüssigkeit 5 gefüllt. Die Elektrodenvorrichtung 1, die auf eventuelle Leckstellen an der Isolierschicht untersucht werden soll, besteht aus einem Elektrodenkabel 6 mit einem langgestreckten, flexiblen Leiter 7, der an einem am distalen Ende des Elektrodenkabels 6 angebrachten Elektrodenkopf 8 angeschlossen ist. Die Aussenseite des Leiters 7 ist mit einer Isolierschicht 9 versehen und dessen Innenseite bildet ein Kanal 10. Der Aussendurchmesser des Organs 2 ist kleiner als der Innendurchmesser des Kanals, so dass das Organ 2 in den Kanal 10 eingeführt werden kann.

Bevor ein Suchen von Leckstellen bei der in einem Patienten implantierten Elektrodenvorrichtung 1 vorgenommen wird, wird ein bekanntes und daher nicht dargestelltes Röntgenuntersuchungsgerät gegen die Brust gerichtet, so dass das gesamte Elektrodenkabel 6 durchleuchtet und auf einem am Untersuchungsgerät angeschlossenen Montior abgebildet wird. Auf dem Monitor kann nun der erfahrene Arzt den Leiter 7 sowie den Elektrodenkopf 8 der Elektrodenvorrichtung 1 sehen.

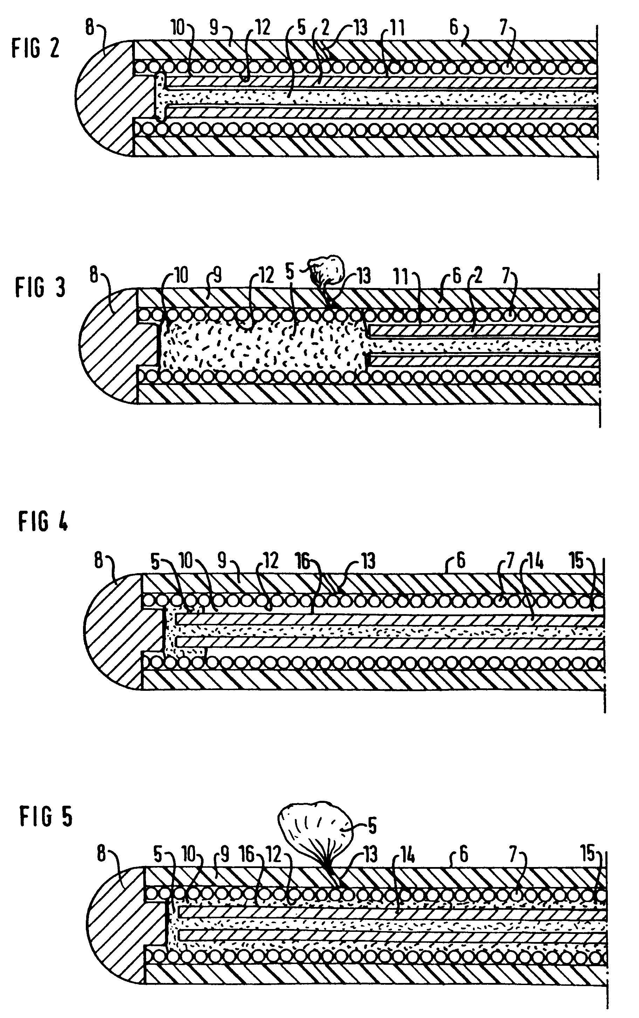

Bei einem Leckstellensuchen bei der implantierten Elektrodenvorrichtung 1 wird wie es in der FIG 1 gezeigt ist, das Organ 2 in den Kanal 10 bis zu dessen distalen Ende hineingeführt. In der FIG 2, die eine Vergrösserung des distalen Endes des Elektrodenkabels 6 in einem Längsschnitt zeigt, ist näher dargestellt, dass das flexible Organ 2 so weit in den Kanal 10 hineingeschoben wird, bis dessen distales Ende mit einem Abstand vom Elektrodenkopf 8, der einer Spaltenöffnung entspricht, zu liegen kommt. Auch das Organ 2 ist hier in einem Längsschnitt gezeigt. Danach wird die Kontrastflüssigkeit 5 mit Hilfe der Spritze 3 über das Organ 2 in den Kanal 10 hineingedrückt. In dieser FIG 2 ist das Organ 2 gezeigt, dessen Aussendurchmesser derart bemessen ist, dass es bei einem im Kanal 10 eingeschobenen Organ 2 nicht möglich ist, dass Kontrastflüssigkeit 5 zwischen der Aussenwand 11 des Organs 2 und der Innenwand 12 des Kanals 10, der von dem Leiter 7 gebildet wird, gefüllt wird. Nun verschiebt der Arzt das Organ 2 im Kanal 10 nach hinten gleichzeitig damit, dass der Kanal 10 vor der Stirnseite des Organs 2 nach und nach mit Kontrastflüssigkeit 5 gefüllt wird, wobei die Füllung derart erfolgt, dass ein radialer Flüssigkeitsdruck im Kanal 10 entsteht, der höher als der das Elektrodenkabel 6 umgebende Druck ist. Luft und eventuelle Schmutzpartikel im Kanal 10 werden nun mit der steigenden Kontrastflüssigkeitssäule zwischen der Aussenwand 11 des Organs 2 und der Innenwand 12 des Kanals 10 hochgedrückt. Wenn die Kontrastflüssigkeit eine Leckstelle 13 an der Isolierschicht, wie es in der FIG. 3 gezeigt ist, erreicht, sickert Kontrastflüssigkeit 5 aus der Leckstelle 13 heraus. Der Arzt, der mit Hilfe der Spritze und der Verschiebung des Organs 2 die Steigungsgeschwindigkeit der Flüssigkeitspegel im Kanal 10 steuert, entdeckt sofort die aus der Leckstelle 13 herausgedrückte Kontrastflüssigkeit 5, wobei der Arzt nun die Leckstelle 13 lokalisieren und registrieren kann. Wenn der Kanal 10 in beschriebener Weise mit Kontrastflüssigkeit 5 gefüllt ist, ist das Suchen von Leckstellen abgeschlossen.

In der FIG. 4 ist gezeigt, dass ein weiteres Organ 14 zum Suchen von Leckstellen in den Kanal 10 des Elektrodenkabels 6 eingeschoben werden kann. Der Aussendurchmesser dieses Organs 14 ist derart bemessen, dass bei einem in dem Kanal 10 eingeschobenen Organ 14 Raum 15 zwischen der Aussenwand 16 des Organs 14 und der Innenwand 12 des Kanals 10 gebildet wird, der mit Kontrastflüssigkeit 5 gefüllt werden kann. Auch dieses Organ 14 wird in den Kanal 10 hineingeschoben, bis dessen distales Ende etwa mit einem Abstand zum Elektrodenkopf 8, der einer Spaltöffnung entspricht, zu liegen kommt. Danach wird, wie es in Verbindung mit der FIG. 2 beschrieben ist, Kontrastflüssigkeit 5 über das Organ 14 in den Kanal 10 hineintransportiert. Im Unterschied zu dem bereits beschriebenen Organ 2 kann das Organ 14 in der beschriebenen Lage stehenbleiben, so dass eine Kontrastflüssigkeitssäule im Raum 15 zwischen der Aussenwand 16 des Organs 14 und der Innenwand 12 des Kanals gebildet wird. Die Kontrastflüssigkeitssäule verschiebt nun Luft und eventuelle Schmutzpartikel vom Kanal 10 vor sich, die daher nicht an einer Leckstelle an der Isolierschicht 9 herausgedrückt werden. Wenn die Kontrastflüssigkeitssäule die Leckstelle an der Isolierschicht erreicht, sickert, wie bereits beschrieben, Kontrastflüssikgeit 5 durch die Leckstelle 13 hindurch. Dies sieht der Arzt, der nun die Lage der Leckstelle am Elektrodenkabel 6 registrieren kann. Wenn der Raum 15 im Kanal 10 mit Kontrastflüssigkeit 5 gefüllt ist, ist das Suchen von Leckstellen abgeschlossen.

Auch mit dem in der FIG 4 und 5 gezeigten Organ 14 kann selbstverständlich eine Kontrastflüssigkeitsfüllung des Kanals 10 durchgeführt werden. Dies erfolgt,indem das Organ nach hinten verschoben wird gleichzeitig damit, dass der Kanal 10 nach und nach mit Kontrastflüssigkeit 5 gefüllt wird.

Aufgrund des Raumes 15, in dem die Kontrastflüssigkeit hochsteigen kann, ist eventuell nicht immer eine deutliche Flüssigkeitssäule, wie es bei dem Organ 2 der Fall ist, das in Verbindung mit den FIG 2 und 3 beschrieben worden ist, gegeben.

Die Anordnung zum Suchen von Leckstellen nach der Erfindung kann selbstverständlich in Verbindung mit anderen denkbaren implantierten Elektrodenvorrichtungen, wie z.B. Defibrillierungselektroden, verwendet werden.

### Bezugszeichenliste

- 1: Elektrodenvorrichtung
- 2, 14: Organ
- 3: Behälter,Spritze
- 4: Schnellverschluss
- 5: Kontrastflüssigkeit, Flüssigkeit
- 6: Elektrodenkabel
- 7: Leiter
- 8: Elektrodenkopf
- 9: Isolierschicht
- 10: Kanal
- 11,16: Die Aussenwand des Organs
- 12: Die Innenwand des Kanals
- 13: Leckstelle
- 15: Raum

## Patentansprüche

1. Anordnung zum Suchen von Leckstellen bei einer Elektrodenvorrichtung, insbesondere einer Herzschrittmacher- oder einer Defibrillierungselektrode, bestehend aus einem Elektrodenkabel mit einem langgestreckten flexiblen Leiter, dessen Aussenseite mit einer Isolierschicht versehen ist und dessen Innenseite einen Kanal bildet, **dadurch gekennzeichnet**, dass die Anordnung ein rohrförmiges flexibles Organ (2, 14) ist, dessen Aussendurchmesser kleiner als der Innendurchmesser des Kanals (10) ist und das in den Kanal (10) des Elektrodenkabels (6) bis zum distalen Ende einschiebbar ist, und dass das proximale Ende des Organs (2, 14) an einem Behälter (3) für Flüssigkeit (5) angeschlossen ist, der einen Überdruck erzeugen kann, so dass die Flüssigkeit (5) durch das Organ (2, 14) und in den Kanal (10) transportiert werden und somit den Kanal (10) derart füllen kann, dass ein radialer Flüssigkeitsdruck im Kanal (10) entsteht, der höher als der das Elektrodenkabel (6) umgebende Druck ist.

2. Anordnung nach Anspruch 1, **dadurch gekennzeichnet**, dass der Aussendurchmesser des rohrförmigen, flexiblen Organs (14) derart bemessen ist, dass bei einem in dem Kanal (14) eingeschobenen Organ (14) Raum (15) zwischen der Aussenwand (16) des Organs (14) und der Innenwand (12) des Kanals (10) gebildet wird, der mit der Flüssigkeit (5) gefüllt werden kann.

3. Anordnung nach Anspruch 1 oder 2, **dadurch gekennzeichnet**, dass die Flüssigkeit (5) durch das Organ (2, 14) transportiert wird, wenn das Organ (2, 14) in dem Kanal (10) des Elektrodenkabels (6) bis zu dessen distalem Ende eingeschoben ist.

4. Anordnung nach Anspruch 1 oder 3, **dadurch gekennzeichnet**, dass der Aussendurchmesser des rohrförmigen, flexiblen Organs (2) derart bemessen ist, dass es bei einem im Kanal (10) eingeschobenen Organ (2) nicht möglich ist, dass Flüssigkeit (5) in den Spalt zwischen der Aussenwand (11) des Organs (2) und der Innenwand (12) des Kanals (10) eindringt.

5. Anordnung nach einem der Ansprüche 1 bis 4, **dadurch gekennzeichnet**, dass die Flüssigkeit (5) ein Kontrastmittel ist.

6. Anordnung nach einem der Ansprüche 1 bis 5, **dadurch gekennzeichnet**, dass der Behälter (3) für die Flüssigkeit eine Spritze ist, mit der die Flüssigkeit (5) über das Organ (2, 14) in den Kanal (10) hineingedrückt wird.

7. Verfahren zur Durchführung eines Suchens von Leckstellen bei einer Elektrodenvorrichtung mit einer Anordnung nach einem der Ansprüche 1, 2, 3, 5 und 6, **dadurch gekennzeichnet**, dass das Organ (14) in den Kanal (10) bis zu dessen distalen Ende hineingeschoben und dass die Flüssigkeit (5) danach in den Kanal (10) transportiert wird und den Raum (15) zwischen dem Organ (14) und der Innenwand (12) des Kanals (10) derart füllt, dass ein radialer Flüssigkeitsdruck im Raum (15) entsteht, der höher als der das Elektrodenkabel (6) umgebende Druck ist.

8. Verfahren zur Durchführung eines Suchens von Leckstellen bei einer Elektrodenvorrichtung mit einer Anordnung nach einem der Ansprüche 1 und 3 bis 6, **dadurch gekennzeichnet**, dass das Organ (2) in den Kanal (10) bis zu dessen distalen Ende hineingeschoben und dass die Flüssigkeit (5) danach in den Kanal (10) transportiert wird, wobei das Organ (2) nach hinten verschoben wird gleichzeitig damit, dass der Kanal (10) vor der Stirnseite des Organs (2) nach und nach mit Flüssigkeit (5) gefüllt wird, wobei die Füllung derart erfolgt, dass ein radialer Flüssigkeitsdruck im Kanal (10) entsteht, der höher als der das Elektrodenkabel (6) umgebende Druck ist.
